# EUROPEAN PATENT SPECIFICATION

(11) **EP 3 071 224 B1**
(45) Date of publication and mention of the grant of the patent: **09.01.2019**
(21) Application number: 14827177.8
(22) Date of filing: 22.11.2014
(51) Int. Cl.: A61K 39/00

(54) **METHOD FOR RESTORING IMMUNE TOLERANCE IN VIVO**
VERFAHREN ZUR IN-VIVO-WIEDERHERSTELLUNG EINER IMMUNTOLERANZ
PROCÉDÉ DE RESTAURATION DE LA TOLÉRANCE IMMUNITAIRE IN VIVO

(30) Priority: 22.11.2013 EP 13194126; 29.01.2014 EP 14153144
(43) Date of publication of application: 28.09.2016
(73) Proprietor: Amarna Holding B.V., 2333 CH Leiden (NL)
(72) Inventor: HAAN, DE, Petrus Theodorus, NL-2343 RR Oegstgeest (NL)
(74) Representative: Life Science Patents B.V.
(86) International application number: PCT/EP2014/075346
(87) International publication number: WO 2015/075213

(56) References cited:
- EP-A1- 2 243 836
- WO-A2-03/045316
- WO-A2-2006/124375
- US-A1- 2002 068 715
- US-A1- 2012 076 808
- JINDAL R M ET AL: "Prevention of diabetes in the NOD mouse by intra-muscular injection of recombinant adeno-associated virus containing the preproinsulin II gene", INTERNATIONAL JOURNAL OF EXPERIMENTAL DIABETES RESEARCH, HARWOOD ACADEMIC PUBLISHERS, US, vol. 2, 1 January 2001 (2001-01-01), pages 129-138, XP002964168, ISSN: 1560-4284

## Description

### Field of the invention

The present description provides an improved method for the restoration of immune tolerance in vivo. In particular, the description relates to the use of a recombinant gene encoding one or multiple auto-antigens or antigenic parts thereof to restore immune tolerance to said antigens in vivo. The description also provides compositions comprising a recombinant gene encoding one or multiple auto-antigens or antigenic parts thereof and uses thereof as a treatment for autoimmune diseases.

### Background of the invention

The immune system consists of an intricate network of tissues, cells, and molecules responsible for responding to cell death, wounding or pathogen infection and maintaining the body's homeostasis. During homeostasis, dying cells that undergo programmed cell death (apoptosis) are removed by specific cells of the immune system, e.g., immature macrophages and dendritic cells, and are replenished by cells descending from tissue-resident stem cells. The cellular components are processed by the immature macrophages and dendritic cells into auto-antigens that are presented on major histocompatibility complex (MHC) molecules to cells of the adaptive immune system (signal 1). Macrophages and dendritic cells therefore represent the main antigen-presenting cells in the body. Antigen presentation takes place in the presence of tolerogenic co-stimulatory molecules (signal 2) such as B7-1 (CD80), B7-H1 (CD274), B7-DC, B7-H3 and B7-H4 and tolerogenic chemokines as well as cytokines (signal 3) such as interleukin 10 (IL10) and transforming growth factor beta. Immature antigen-presenting cells carry chemokine receptors CCR1, CCR2, CCR5, CCR6 and CXCR1; antigen receptors of the C-type lectin family such as DEC-205, the dendritic cell immunoreceptor, dectin-2 and C-type lectin receptor 1; the macrophage mannose receptor and Fc receptors for immunoglobulins such as the Fc gamma receptor and Fc epsilon receptor; toll-like receptors; integrins and heat shock protein receptors. In addition, immature antigen-presenting cells are characterized by having high levels of intracellular MHC, whereas they have low levels of B7-2 (CD86), CD40, CD25 and CD83 on their surface. As a result of these homeostatic cell-cell interactions the immune system is in the immune tolerance state.

Following wounding or pathogen infection, injured or infected cells undergo necrosis and release danger-associated or pathogen-associated molecular patterns, respectively. These molecular patterns induce local tissue inflammation at the site of injury or infection. Necrotic cells are removed by antigen-presenting cells that subsequently mature and they present the processed (self) antigens to cells of the adaptive immune system in the presence of proinflammatory signals 2 and 3. During their conversion from immature to mature cells, antigen-presenting cells undergo a number of phenotypical and functional changes.

Antigen-presenting cell maturation involves: (i) redistribution of MHC molecules from intracellular endocytic compartments to the cell surface; (ii) down-regulation of antigen internalization; (iii) increase in the surface expression of co-stimulatory molecules such as B7-2, CD40 and B7-H2 (CD275) (signals 2); (iv) morphological changes (e.g., formation of dendrites); (v) cytoskeleton re-organization; (vi) secretion of chemokines such as CCL17, CCL18, CCL22 and cytokines such as IL3, IL4, IL5, IL6, IL12, IL13, IL14, interferon alpha, interferon beta, interferon gamma and tumor necrosis factor alpha (signals 3) and (vii) surface expression of adhesion molecules and chemokine receptors such as CXCR4 and CCR7.

Mature antigen-presenting cells are characterized by having low levels of Fc receptors, intercellular adhesion molecule-1 and CD58 on their surface. Cells of the adaptive immune system, mainly consisting of T- and B-cells activated by interacting with the mature antigen-presenting cells, now temporarily break the immune tolerance and destruct/clear the injured or infected cells in an antigen-specific manner. After a normal wound repair response, thus in the absence of the danger-associated or pathogen-associated molecular patterns, the immune tolerance is restored, again in an antigen-specific manner.

In patients with degenerative diseases, such as dystrophic diseases and chronic viral infections, at a certain point in time specific tissues/organs (brain, muscle, blood, liver, eye, skin etc.) start to accumulate necrotic cells. The necrotic cells induce non-resolving inflammation, wherein chronic activation of cells of the adaptive immune system leads to a continuous and local breaking of the immune tolerance and to a destruction of cells of the inflamed tissue in an antigen-specific manner. The activated cytotoxic T lymphocytes destruct their target cells by inducing cell necrosis. The necrotic cells on their turn further enhance tissue inflammation. In addition, chronic inflammation results in amyloid plaque formation and tissue scarification due to collagen deposition. It has been generally accepted that this auto-antigen-specific chronic stimulation of the adaptive immune response is the major driver of the tissue/organ destruction (Nathan C. and Ding A., Cell 140: 871-882, 2010).

Examples of genetic disorders resulting in a degenerative disease are neurological diseases such as Huntington disease and the familial forms of Alzheimer's dementia and Parkinson disease; the familial muscular dystrophies such as Duchenne muscular dystrophy, Becker muscular dystrophy, Miyoshi myopathy, Limb-girdle muscular dystrophy, Congenital muscular dystrophy, Distal muscular dystrophy, Emery-Dreyfuss muscular dystrophy, Fascio-scapulohumeral muscular dystrophy, Myotonic muscular dystrophy and Oculopharyngeal muscular dystrophy; ophthalmological dystrophies such as Retinitis pigmentosa, Leber's congenital amaurosis, Stargardt macular dystrophy, Achromatopsia, Retinoschisis and Vitelliform macular dystrophy.

In sporadic cases, these diseases are acquired diseases. This means that in genetically predisposed individuals at a certain point in time, unknown environmental stimuli, mimicking danger- or pathogen-associated molecular patterns, initiate the process of nonresolving inflammation of specific tissues. In these cases the diseases are named autoinflammatory or autoimmune diseases.

With our increased knowledge in human immunology the list of autoimmune diseases is expanding rapidly and currently includes, but is not limited to, neurological diseases such as Alzheimer's dementia, Amyotrophic lateral sclerosis, Autism, Bipolar disorder, Depression, Epilepsy, Narcolepsy, Lyme disease, Multiple system atrophy, Multiple sclerosis, Myasthenia gravis, Neuromyelitis optica, Parkinson disease and Schizophrenia; metabolic diseases such as Type 1 diabetes, Type 2 diabetes, Hashimoto's thyroiditis and Obesity; muscular diseases such as Polymyositis, Dermatomyositis and inclusion body myositis; ophthalmological diseases such as autoimmune retinopathy, Age-related macular degeneration, Glaucoma and Uveitis; gastro-enteric diseases such as Celiac disease and Inflammatory bowel diseases including Crohn's disease and Ulcerative colitis; dermatological diseases such as Psoriasis, Scleroderma, Sjögren's syndrome and Vitiligo; cardio-vascular diseases such as Atherosclerosis, Cardiomyopathy and Coxsackie myocarditis; orthopedic diseases such as Rheumatoid arthritis, Rheumatic fever and Lupus erythematosus; and pulmonary diseases such as Chronic obstructive pulmonary disease and asthma. Furthermore, Amyloidosis, Autoimmune hepatitis, Chagas disease, Endometriosis, Goodpasture's syndrome, Hashimoto's encephalitis, Idiopathic thrombocytopenic purpura, Kawasaki syndrome, Meniere disease, Neutropenia, Restless legs syndrome and Thrombocytopenic purpura are autoimmune diseases.

Current treatments for patients with a degenerative or dystrophic disease non-specifically suppress inflammation and immunity, and thus only alleviate the symptoms and delay the progression to disabling stages. Furthermore, long term use of immuno-suppressing medication coincides with often severe adverse side effects and enhances the risk of developing autoimmune processes in other tissues.

There is, therefore, an urgent need for alternative treatments that specifically inhibit tissue destruction by activated cells of the adaptive immune system, leaving the general immune response unaffected. In principle, this should be feasible since autoimmune diseases are acquired diseases. Restoring immune tolerance to the major auto-antigens involved in the autoimmune tissue destruction has been a longstanding goal in autoimmunity research. This has been attempted by administration of the auto-antigens or peptide fragments derived thereof to patients. However, due to the fact that these proteins or peptides are not efficiently delivered to the proper antigen-presenting cells, that they lack the proper accompanying signals for reverting the immune response and/or that they are rapidly degraded in the body, such approaches have been adopted with limited success.

An efficient way to instruct cells of the immune system to suppress an autoimmune response is to let them produce the auto-antigens involved in the autoimmune disease by introducing the antigen-encoding genes into these cells. Transgenic mice expressing myelin basic protein (MBP) in the liver are protected from MBP-induced experimental autoimmune encephalitis (EAE), a neurological disease in mouse with symptoms that resemble multiple sclerosis in humans. Transgenic mice expressing MBP in the skin and thymus however, are not protected from developing EAE, indicating that the liver is an important tolerogenic organ (Lueth S. et al., The Journal of Clinical Investigation 118: 3403-3410, 2008). In a therapeutic setting, the auto-antigen-encoding therapeutic genes can be administered as naked molecules or as nucleic acids packaged in lipid and/or proteinaceous compounds. In mice with myelin oligodendrocyte glycoprotein (MOG)-induced EAE model, injection of an expression plasmid encoding MOG or MBP reduced the clinical and histopathological signs of EAE in both prophylactic and therapeutic settings (Garren H., Expert Opinion Biological Therapy 8: 1539-1550, 2008; Fissolo N. et al., Journal of Neuroinflammation 9: 139-152, 2012; US20100160415). In non-obese diabetes (NOD) mice, the most commonly used animal model for type 1 diabetes in humans, injection of expression plasmids encoding preproinsulin or glutamic acid decarboxylase 65 (GAD65), the major auto-antigens involved in autoimmune destruction of the pancreatic insulin-producing cells, results in both prevention and suppression of autoimmune diabetes (Johnson M.C. et al., Human Vaccines 7: 27-36, 2011; Guan Y. et al., Diabetes Research Clinical Practice 95: 93-97, 2012). This DNA vaccination approach however, only works in mice. In all other mammalian species including humans, cells do not take up and express extracellular nucleic acids.

Since viruses evolved to deliver and express their genetic information into host target cells, viral vectors are currently the most effective gene delivery vehicles. Besides gene therapy applications viral vectors have been shown to be highly effective in modulating immune responses in animal models of autoimmune diseases.

WO 03/045316 A2 discloses a vector comprising a nucleic acid sequence encoding an auto-antigen under the transcriptional control of SV40 early or late promoter for treating or preventing an autoimmune disease.

EP 2243836 A1 describes the use of SV40 particles comprising a nucleic acid sequence encoding an autoantigen for use in the treatment of an autoimmune disease.

In mice with Rheumatoid arthritis induced by subcutaneous administration of type II collagen, the disease progression was halted by intravenous administration of a lentiviral vector encoding type II collagen (Eneljung T. et al., Clinical and Developmental Immunology 2013: 11, 2013).

In NOD mice that have been administered with adeno-associated virus (AAV) or lentiviral vectors encoding preproinsulin or GAD65 immune tolerance to the pancreatic insulin-producing cells was efficiently restored and the development of Type 1 diabetes was prevented (Johnson M.C. et al., Human Vaccines 7: 1-10, 2011; Jindal R.M. et al., International Journal of Experimental Diabetes Research 2: 129-138, 2001; Dresch C. et al., Journal of Immunology 181: 4495-4506, 2008; Han G. et al., Journal of Immunology 174: 4516-4524, 2005; Han G. et al., Immunology Letters 115: 110-116, 2008; Xu B. and Scott D.W., Clinical Immunology 111: 47-52, 2004). Implantation of bone marrow-derived stem cells or hepatic cells transduced ex vivo with lentiviral vectors encoding the major MOG epitopes in mice, protected the treated animals from MOG-induced EAE (Hoffman B.E. and Herzog R.W., The Journal of Clinical Investigation 118: 3271-3273, 2008; De Andrade Pereira D. et al., Gene Therapy 20: 556-566, 2013). Intrathymical delivery of MOG using a lentiviral vector induced central immune tolerance to MOG, which prevented mice from developing EAE upon challenge with MOG (Marodon G. et al., Blood 108: 2972-2978, 2006). The progression of EAE in mice that received MOG first could, however, not be stopped using this central tolerization approach (Siatskas C. et al., Molecular Therapy 20: 1349-1359, 2012). This study demonstrates that auto-antigens need to be expressed and exposed to antigen presenting cells in the periphery preferably in tolerogenic organs, in order to revert the pre-existing immune response into an immune tolerance response.

These and other animal studies have demonstrated that an active and specific immune tolerance response can be induced against a self-protein when the gene encoding the self-protein is expressed ectopically under transcriptional control of liver-specific promoters derived from host genes, such as the albumin, the DC190, the alpha anti-trypsin (AAT), the C-reactive protein (CRP) or the dendritic cell-specific transmembrane protein (DC-STAMP) promoters (Follenzi A. et al., Blood 103: 3700-3709, 2004; Siatskas C. et al., Molecular Therapy 20: 1349-1359, 2012; Lueth S. et al., The Journal of Clinical Investigation 118: 3403-3410, 2008; De Andrade Pereira D. et al., Gene Therapy 20: 556-566, 2013; Hoffman B.E. and Herzog R.W., The Journal of Clinical Investigation 118: 3271-3273, 2008). In addition, liver-specific expression of coagulation factors VIII or IX introduced by AAV or lentiviral vectors efficiently restored immune tolerance to the coagulation factors in dogs and mice with pre-existing immunity to these factors (Siatskas C. et al., Molecular Therapy 20: 1349-1359, 2012; Finn J.D. et al., Blood 116: 5842-5848, 2010). However, recent clinical gene therapy studies conducted demonstrated that the organ-specific and host-derived promoters used to control transcription of the therapeutic genes are not potent enough to yield therapeutic levels of self-proteins in the treated patients (Nathwani A.C. et al., New England Journal of Medicine 365: 2357-2365, 2011).

Moreover, ectopic expression of genes encoding self-proteins under transcriptional control of strong constitutive viral promoters such as the cytomegalovirus (CMV) immediate early promoter or retroviral long terminal repeat promoters leads to the induction of an adaptive immune response directed against the self-protein (Follenzi A. et al., Blood 103: 3700-3709, 2004; Ko H.J. et al., Autoimmunity 44: 177-187, 2011). For example, it has been found that macaques develop an autoimmune anemia upon gene therapy with adeno-associated virus vectors encoding erythropoietin (Gao G. et al., Blood 103: 3300-3302, 2004; Chenuaud P. et al., Blood 103: 3303-3304, 2004). Erythropoietin was over-expressed in macaques under transcriptional control of the cytomegalovirus immediate early promoter or a doxycyclin-inducible promoter derived from the HIV-1 long terminal repeat. These animals were found to develop an adaptive immune response against endogenous erythropoietin or cells producing erythropoietin. So immune therapy wherein auto-antigens are over-expressed may induce rather than cure an autoimmune disease.

Although viral vector-based tolerization approaches to treat degenerative diseases using organ-specific and host-derived promoters driving expression of auto-antigens look promising, it has become obvious from the above described prior art that there is an unmet need for treatments that restore immune tolerance to said auto-antigens.

### Summary of the invention

The above objects have been met as described herein in that a method is provided for restoring immune tolerance in vivo. The description provides the use of a recombinant gene comprising a heterologous DNA sequence harboring the coding domain of one or multiple auto-antigens or antigenic parts thereof and wherein the heterologous DNA is expressed in a target cell under transcriptional control of a full-length polyomaviral early and late promoter, also often referred to as the polyomaviral intergenic region. This leads to restoring immune tolerance to said auto-antigens in vivo. It is preferred that the polyomaviral intergenic region is the SV40 intergenic region or full-length SV40 early and late promoter as disclosed in SEQ ID NO: 12. It is also preferred that a promoter according to SEQ ID NO: 1 is used.

The present description therefore provides a method for restoring immune tolerance in vivo. The description relates to the use of a recombinant gene encoding auto-antigens or antigenic parts thereof for restoring immune tolerance to said auto-antigens in vivo, under transcriptional control of polyomaviral early and/or late promoters. The description also relates to the use of recombinant polyomaviral gene delivery vector particles, such as simian virus 40 (SV40) viral vector particles encoding one or multiple auto-antigens or antigenic parts thereof under transcriptional control of polyomaviral early and late promoter such as the full length SV40 early and late promoter, for restoring immune tolerance to said auto-antigens in vivo. The description also relates to compositions comprising recombinant genes or polyomaviral vectors and uses thereof as treatment for degenerative or dystrophic diseases.

It is preferred that a full-length polyomaviral early and late promoter is used, preferably the SV40 early and late promoter, (SEQ ID NO: 12 or SEQ ID NO: 1).

### Legend to the figure.

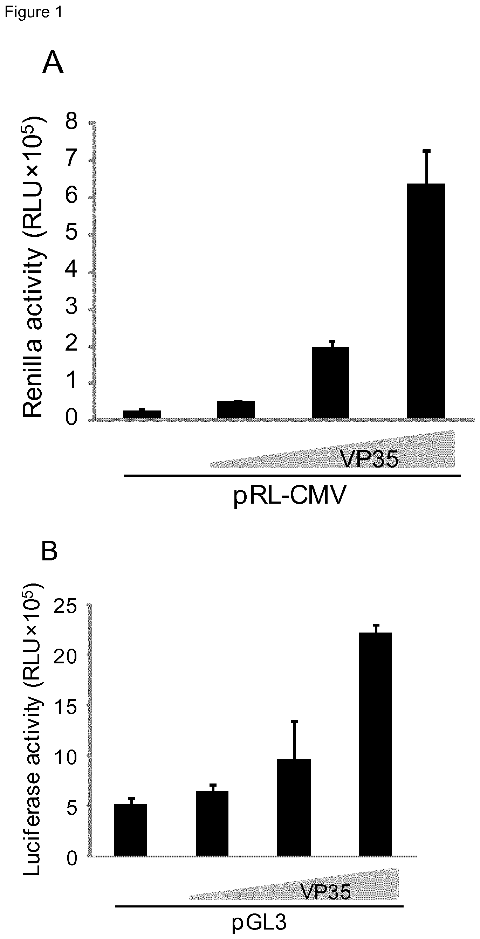

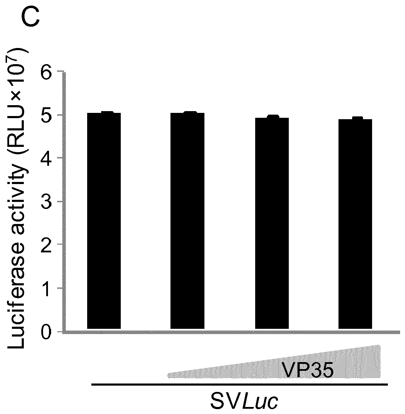

Figure 1: Graphs showing the relation between suppression of RNA interference (0, 10, 50 and 100 nanograms of VP35) and expression of luciferase under the control of three different promoters: Panel A: CMV immediate early promoter (pRL-CMV), Panel B: SV40 early promoter (pGL3) and Panel C: the full-length SV40 early and late promoter (SV*Luc*, SEQ ID NO: 12).

### Detailed description of the invention

The present inventor surprisingly found that human peripheral blood mononuclear cells (PBMC's) secreted tolerogenic cytokines upon transfection with an expression plasmid encoding an antigen under the control of a full-length polyomaviral early and late promoter.

The expression plasmids were constructed wherein the expression of firefly luciferase and myelin oligodendrocyte glycoprotein (MOG) was under the transcriptional control of the polyomaviral intergenic region. It was found that immune tolerance was induced against these antigens. This result shows that we were able to induce tolerance against auto-antigen as well as non-self antigen by expressing these antigens under the control of the polyomaviral intergenic region, comprising the full-length polyomaviral early and late promoter.

This is in contrast to a prejudice that viral promoters induce an adaptive immune response to a transgene product expressed under their transcriptional control. Without wanting to be bound by theory, we hypothesized that the main reason why viral promoters in general induce an immune response in vivo is that these viral promoters induce RNA interference, in which the messenger RNA molecules encoding the transgene products are degraded, resulting in the accumulation of short double stranded RNA molecules in the cytoplasm. Double stranded RNA molecules are highly effective inductors of inflammatory responses.

The capability of promoters to induce RNA interference in cells can be tested in an assay where an expression plasmid encoding a reporter protein under transcriptional control of a promoter to be tested is transferred to cells in the presence or absence of an expression plasmid encoding a viral suppressor of RNA interference, such as Ebola virus VP35 protein. In this assay, cells containing expression plasmids with promoters that induce RNA interference accumulate low amounts of reporter protein in the absence of a viral suppressor of RNA interference. Cells containing expression plasmids with promoters that do not induce RNA interference accumulate high amounts of reporter protein independent of the presence or absence of a viral suppressor of RNA interference (Figure 1).

Similar to the cytomegalovirus immediate early promoter and the retroviral long terminal repeat promoters the SV40 early promoter present in the expression plasmids described in US20100160415 and in the commercially available expression plasmids such as pGL3, pSG5, pRC/RSV are potent inducers of RNA interference in cells and thus of adaptive immune responses in vivo. Said commercially available SV40 early promoters comprise only a part of the SV40 intergenic region including the domains with binding sites for transcription factors, the origin of replication and the transcriptional enhancer sequences. A number of reports name the potential use of unspecified SV40 promoters (WO 03045316 A2; WO 2006124375 A2; US 2002068715 A1; US 2012076808 A1) for restoring immune tolerance in vivo. The unspecified SV40 promoters comprise part of the SV40 intergenic region and are not equivalent to the full-length SV40 early and late promoter of the present description (SEQ ID NO: 12, SEQ ID NO: 1).

In one preferred aspect, the method as described herein includes the use of the SV40 intergenic region comprised between nucleotides 5171 and 334 in the SV40 genome published under the reference sequence NC_001669 at US National Center for Biotechnology Information (NCBI) (SEQ ID NO: 12). This region contains the early and late promoter with the binding sites for transcription factors, the origin of replication, all transcriptional enhancer sequences, the origin-of-replication, the encapsidation signals and a DNA secondary structure that is responsible for specific and efficient binding of the host DNA-dependent RNA polymerase (Vogel F. et al., Gene 16: 331-334, 1981). The inability to induce RNA interference in a mammalian cell makes a polyomaviral full-length early and late promoter ideally suited for inducing immune tolerance in vivo (See example 15).

Since the full length SV40 early and late promoter sequence also comprises the viral origin-of-replication and encapsidation signals, the expression plasmid was used for the production of replication-deficient or replication-defective SV40 gene delivery vector particles (denoted SV*Luc*). Human PBMC's transduced with SV*Luc* particles also secrete tolerogenic cytokines. In addition, the macrophages and dendritic cells present in the transduced mononuclear cells carried tolerogenic co-stimulatory molecules and chemokine receptors on their surface showed high levels of intracellular MHC, indicating that these cells remained immature. In contrast, human PBMC's transduced with a recombinant SV40 vector encoding the firefly luciferase under transcriptional control of the cytomegalovirus immediate early promoter, secreted pro-inflammatory cytokines. The macrophages and dendritic cells present in the transduced cells carried proinflammatory co-stimulatory molecules and chemokine receptors on their surface and showed low levels of intracellular MHC, indicating that these cells maturated. It was concluded from this surprising result that although the entire full-length SV40 intergenic region is of viral origin, it is capable of inducing immune tolerance. Since polyomaviral gene delivery vectors, including SV40 vectors, are immunologically inert in humans, said vectors have the unique capability of inducing an active immune tolerance response to a self-protein encoded by the vector. Replication-deficient or replication defective polyomaviral vectors, in which the early or late coding domain has been replaced with a gene encoding an auto-antigen and where the auto-antigen is expressed in transduced cells under transcriptional control of the entire full-length polyomaviral intergenic region, are highly efficient in restoring auto-antigen-specific immune tolerance in patients with a degenerative disease, such as an autoimmune disease.

SV40 belongs to the Polyomaviruses, which comprises a family of non-enveloped DNA (double stranded) viruses with icosahedral capsids. SV40 has a circular genome with a length of 5.25 kilo base pairs and consists of two regulatory regions: the promoter/origin region and the polyadenylation region, as well as the early and late regions. The SV40 early promoter controls transcription of the early region, which encodes the small and large T antigens. The late promoter controls transcription of the late region, which encodes the viral capsid proteins (VP1, VP2 and VP3).

In polyomaviral vectors, T antigen-specific sequences have been deleted, not only for rendering the vectors replication-incompetent, but also to completely eliminate their immunogenicity. Polyomaviral vectors derived from SV40 have been produced and tested, in which the therapeutic genes or nucleic acids are expressed in target cells under transcriptional control of the viral early promoter. Said vectors have been used for gene transfer into a wide variety of human tissues and cell types, for example, bone marrow (Rund D. et al., Human Gene Therapy 9: 649-657, 1998), the liver (Strayer D.S. and Zern M.A., Seminars in Liver Disease 19: 71-81, 1999) and dendritic cells (Vera M. et al., Molecular Therapy 12: 950-959, 2005).

Polyomaviral vectors, such as SV40, are known to infect non-dividing as well as dividing cells. Since SV40 is a macaque polyomavirus that does not occur in the human population and SV40 vectors are replication deficient, SV40 vectors are non-immunogenic in humans and allow for repeated administration to the same individual (Strayer D.S. and Zern M.A., Seminars in Liver Disease 19: 71-81, 1999).

The present description relates to the use of a recombinant gene comprising a heterologous DNA encoding one or multiple auto-antigens or antigenic parts thereof and an entire full-length polyomaviral intergenic region, for restoring immune tolerance to said auto-antigens in vivo.

As described herein, the heterologous DNA refers to a DNA sequence that codes for one or multiple auto-antigens or antigenic parts thereof. The term auto-antigen as used herein is used for a protein produced by cells of a patient suffering from a degenerative or dystrophic disease that is specifically targeted by the patients' adaptive immune system and that as a result is involved in the destruction of said cells in the course of the disease. A suitable example of a degenerative or dystrophic disease is an auto-immune disease.

The adaptive immune system comprises humoral and cellular components. The humoral adaptive immune system consists of antibodies produced by B lymphocytes. The cellular adaptive immune system mainly consists of T lymphocytes. Traditionally, the classification of a disease as autoimmune has been based on the detection of antibodies denoted autoantibodies that recognize and bind to auto-antigens.

Methods to identify auto-antigens that are involved in autoimmune tissue destruction are based on detection of components of the cellular adaptive immune system including but not limited to: i) construction of an expression cDNA library of the tissue affected by a degenerative or dystrophic disease; ii) expression of the cDNA's in human cells; iii) bring said human cells into contact with PBMC's obtained from patients with said degenerative or dystrophic disease; iv) measure the amounts of pro-inflammatory cytokines produced by activated lymphocytes present in the PBMC population. The cDNA molecules that induce the production of pro-inflammatory cytokines in PBMC's from patients with a degenerative or dystrophic disease, encode a major auto-antigen involved in the autoimmune tissue destruction.

An alternative method to identify auto-antigens that are involved in autoimmune tissue destruction is also based on detection of components of the cellular adaptive immune system. Said alternative method includes: i) construction of an expression cDNA library of the tissue affected by a degenerative disease; ii) expression of the cDNA's in PBMC's obtained from patients with said degenerative disease; iii) measure the amounts of pro-inflammatory cytokines produced by activated T lymphocytes present in the PBMC population. The cDNA molecules that induce the production of pro-inflammatory cytokines in PBMC's from patients with a degenerative disease, encode a major auto-antigen involved in the autoimmune tissue destruction.

In order to further confirm that the identified auto-antigen is a major driver of autoimmune destruction, the protein encoded by the cDNA is produced in cells using standard techniques well known in the art. Purified protein is subsequently injected into a mammal together with a strong adjuvant such as Freund's complete adjuvant. The injected mammal will subsequently be monitored for the development of degenerative or dystrophic disease symptoms. The mammals that develop degenerative or dystrophic disease symptoms have been administered with a major auto-antigen involved in the autoimmune cell destruction.

For a number of autoimmune diseases the major auto-antigens involved in the autoimmune cell destruction are known. For the majority of degenerative or dystrophic diseases however, said auto-antigens await to be identified using the confirmatory methods described above or using other immunological methods known to those skilled in the art. Examples of auto-antigens involved in autoimmune disease are: the neurofilament light (NFL) chain and alpha-actinin involved in Amyotrophic lateral sclerosis; forminotransferase cyclodeaminase involved in Autoimmune hepatitis; tissue transglutaminase involved in Celiac disease; collagen type IV involved in Goodpasture's syndrome; thyroglobulin involved in Hashimoto's thyroiditis; platelet-specific glycoproteins IIb/IIIa, Ib/IX, Ia/IIa and IV involved in Idiopathic thrombocytopenic purpura; endothelial cell growth factor involved in Lyme disease; the acetylcholine receptor involved in Myasthenia gravis; hypocretin-1/orexin involved in Narcolepsy; aquaporin 4 involved in Neuromyelitis optica; RNA-induced silencing complex (RISC) components involved in Systemic lupus erythematosus; myelin oligodendrocyte glycoprotein (MOG) and myelin basic protein (MBP) involved in Multiple sclerosis; Golgi SNAP receptor complex member 1 (GOSR1) involved in Obesity and Obesity-induced insulin resistance (Type 2 diabetes); keratin involved in Psoriasis; type II collagen involved in Rheumatoid arthritis; proinsulin and glutamic acid decarboxylase 65 involved in type 1 diabetes; Elastin and beta-2-glycoprotein I involved in Atherosclerosis and Chronic obstructive pulmonary disease; alpha-fodrin involved in Sjögren's syndrome; the glutamate receptor type 3 (GluR3) involved in Epilepsy; histidyl-tRNA-synthetase and myosin-binding C protein in muscular dystrophies; cochlin and beta-tectorin in Meniere disease; arrestin, type I collagen and interphotoreceptor retinoid-binding protein in ophthalmological dystrophies and autoimmune diseases.

The polyomavirus intergenic region sequence resides between the early and late coding regions and comprises the full-length polyomaviral early and late promoter. to the description also provides the use of the SV40 intergenic region on the SV40 genomic DNA residing between the early and late coding regions and comprising the full-length SV40 early and late promoter (SEQ ID NO: 12 and SEQ ID NO: 1 comprise the full-length early and late SV40 promoter).

By "promoter" is meant a sequence of nucleotides from which transcription may be initiated of DNA operably linked downstream (i.e., in the 3' direction on the sense strand of double stranded DNA). "Operably linked" means joined as part of the same nucleic acid molecule, suitably positioned and oriented for transcription to be initiated from the promoter. DNA operably linked to a promoter is "under transcriptional control" of the promoter.

Generally speaking, those skilled in the art are well able to construct a recombinant gene comprising a heterologous DNA encoding one or multiple auto-antigens or antigenic parts thereof and full-length polyomaviral early and late promoters. For further details see, for example, Molecular Cloning: a Laboratory Manual: 2nd edition, Sambrook et al., Cold Spring Harbor Laboratory Press, 1989.

It is also described herein that the recombinant gene is packaged in recombinant polyomaviral gene delivery vector particles, encoding one or multiple auto-antigens or antigenic parts thereof under transcriptional control of the full-length polyomaviral early and late promoter, for restoring immune tolerance to said antigens in vivo.

It is also described herein that the recombinant gene is packaged in recombinant primate polyomaviral gene delivery vector particles, such as a simian polyomavirus, more in particular an SV40, Simian virus 12 (SV12), Lymphotropic polyomavirus, African green monkey polyomavirus or Chimpanzee polyomavirus gene delivery vector particles.

Immune tolerance can be induced in a subject by ectopic expression of the recombinant gene as described herein, or by recombinant polyomaviral gene delivery vector particles as described herein. The term ectopic expression used herein refers to the accumulation of one or multiple auto-antigens or antigenic parts thereof in cell types that under normal conditions do not accumulate said proteins or antigenic parts thereof. Induction of immune tolerance can be experimentally demonstrated as follows: i) human PBMC's are transfected with a recombinant gene as described herein, or transduced with recombinant primate polyomaviral gene delivery vector particles as described herein, encoding one or multiple auto-antigens or antigenic parts thereof; ii) the transfected or transduced PBMC's secrete tolerogenic cytokines such as IL10 and transforming growth factor beta, whereas the amounts of proinflammatory cytokines such as IL3, IL4, IL5, IL6, IL12, IL13, IL14, interferon alpha, interferon beta, interferon gamma and tumor necrosis factor alpha remain low; iii) the macrophages and dendritic cells isolated from the transduced PBMC's remain immature. The maturation stage of the macrophages and dendritic cells can be estimated by measuring the amounts and localization of specific maturation markers such as B7-1, B7-2, CD40, CD25, CD58, CD83, Fc receptors and intercellular adhesion molecule-1.

Also described herein is the use of said recombinant genes or polyomaviral gene delivery vector particles to restore immune tolerance in vivo. Restoration of immune tolerance can be experimentally demonstrated as follows: animal models of autoimmune disease are administered with a recombinant gene or vector as described herein, encoding one or multiple auto-antigens or antigenic parts thereof that are involved in the autoimmune cell destruction in said animal models. As a result the immune tolerance to the auto-antigens is restored and the progression of the autoimmune disease symptoms in the treated animals stops.

The description also provides compositions comprising recombinant genes or polyomaviral vectors and uses thereof as treatment for autoimmune diseases. The pharmaceutical composition may comprise a therapeutically effective amount of one or more recombinant genes, or one or more polyomaviral vectors such as SV40 vectors, and a pharmaceutically acceptable carrier or diluent. The pharmaceutical compositions as described herein can be formulated in any suitable form for administration to the individual in need thereof. Such formulations may be in any form for administration such as topical, oral, parenteral, intranasal, intraocular, intravenous, intramuscular, intralymphatic, subcutaneous or transdermal administration.

The pharmaceutical compositions as described herein generally comprise a buffering agent, an agent that adjusts the osmolarity thereof, and optionally, one or more pharmaceutically acceptable carriers, excipients and/or additives as known in the art. Supplementary active ingredients may also be incorporated into the compositions as described herein. The carrier may be a solvent or dispersion medium containing, for example, water, ethanol, polyol (for example, glycerol, propylene glycol, liquid polyethylene glycol, and the like), suitable mixtures thereof, and vegetable oils. The correct fluidity may be maintained by the use of a coating, such as lecithin, by the maintenance of the required particle size in the case of dispersion and by the use of surfactants.

In summary, the description provides the following:
A vector comprising a nucleic acid sequence encoding an antigen under the transcriptional control of a polyomaviral early and/or late promoter, for use in inducing an immune tolerance towards the antigen in a host organism. This may also be stated as that the method as described herein provides a method for inducing an immune tolerance towards the antigen in a host organism, wherein a vector is administered to the host organism, wherein the vector comprises a nucleic acid sequence encoding an antigen under the transcriptional control of a full-length polyomaviral early and late promoter.

It is preferred that the antigen is under the control of a full-length polyomaviral early and late promoter, such as the full-length SV40 early and late promoter according to SEQ ID NO: 12 or SEQ ID NO: 1.

Advantageously, the antigen in the above mentioned method is an auto-antigen. This allows for the treatment of a number of auto-immune diseases. Advantageously, the auto-antigen is selected from the group consisting of the neurofilament light (NFL) chain, alpha-actinin, beta-2 glycoprotein I, forminotransferase cyclodeaminase, tissue transglutaminase, type IV collagen, thyroglobulin, platelet-specific glycoproteins IIb/IIIa, Ib/IX, Ia/IIa and IV, endothelial cell growth factor, the acetylcholine receptor, aquaporin 4, RNA-induced silencing complex (RISC) components, myelin oligodendrocyte glycoprotein, myelin basic protein, the Golgi SNAP receptor complex member 1 (GOSR1), keratin, type II collagen, proinsulin and glutamic acid decarboxylase 65, elastin, alpha-fodrin, the glutamate receptor type 3 (GluR3), histidyl-tRNA-synthetase, myosin-binding C protein, cochlin, beta-tectorin, arrestin, type I collagen and interphotoreceptor retinoid-binding protein.

The term "antigen" may relate to any substance (as an immunogen or a hapten) that is capable of eliciting an immune response either alone or after forming a complex with a larger molecule for instance a protein and that is capable of binding with a product (as an antibody or T cell) of the immune response. It is preferred that the antigen is an auto-antigen. The term "auto-antigen" is used herein to indicate an antigen derived from the body itself as opposed to non-self antigens which are foreign to the body in which they elicit an immune response. The term "antigen" or "auto-antigen" should not be so narrowly construed as that it is required to consist of a full-length antigen such as a full-length protein, glycoprotein or polysaccharide. It suffices when the antigen consists of an antigenic part or an immunogenic part of the full-length auto-antigen. For instance, if only part of a full-length antigen or auto-antigen is used, it suffices when the part comprises an epitope against which the immune tolerance is to be induced. For general purposes, a fragment of at least 6 amino acids, such as 7, 8, 9, 10, 11, 12, 13, 14, 15 or more amino acids suffices. More preferred is the use of longer fragments, most preferred is the use of the full length antigen comprising many if not all epitopes.

It is also described herein that the auto-antigen is hypocretin-1 or orexin.

Methods as described herein may be advantageously employed in the treatment of an auto-immune disease selected from the group consisting of neurological diseases, Alzheimer's dementia, Amyotrophic lateral sclerosis, Bipolar disorder, Depression, Epilepsy, Lyme disease, Multiple system atrophy, Multiple sclerosis, Myasthenia gravis, Narcolepsy, Neuromyelitis optica, Parkinson disease, Schizophrenia, metabolic diseases, diabetes, Type 2 diabetes, Hashimoto's thyroiditis, Obesitas, ophthalmological diseases, Age-related macular degeneration, Glaucoma, Uveitis, gastro-enteric diseases, Inflammatory bowel diseases, Crohn's disease, Ulcerative colitis, dermatological diseases, Psoriasis, Scleroderma, Vitiligo, cardio-vascular diseases, Atherosclerosis, Cardiomyopathy, Coxsackie myocarditis, Rheumatoid arthritis and Rheumatic fever.

Additionally, the auto-immune disease may be selected from Type-1 diabetes, Retinitis pigmentosa, Leber's congenital amaurosis, Stargardt macular dystrophy, Achromatopsia, Retinoschisis and Vitelliform macular dystrophy, orthopedic dieases, lupus erythematosus, muscular diseases, Polymyositis, Dermatomyositis, Inclusion body myositis, Duchenne muscular dystrophy, Becker muscular dystrophy, Miyoshi myopathy, Limb-girdle muscular dystrophy, Congenital muscular dystrophy, Distal muscular dystrophy, Emery-Dreyfuss muscular dystrophy, Fascio-scapulohumeral muscular dystrophy, Myotonic muscular dystrophy and Oculopharyngeal muscular dystrophy.

In yet another method as described herein, the host is human.

A particularly advantageous use includes administering to the host a vector comprising a full-length polyomaviral early and late promoter and a nucleic acid encoding an antigen wherein the expression of the antigen is under the transcriptional control of the full-length polyomaviral early and late promoter according to SEQ ID NO: 12 or SEQ ID NO: 1.

Vectors as described herein may be administered intravenously. Particularly preferred is a replication-defective SV40 vector.

The technology as described herein will now be further described with reference to the following examples:

### Examples

### Example 1: Construction of a replication deficient SV40 destination vector (pSVac-dest).

Eight oligonucleotides were designed:
WdV101:CCGCTCGAGTTGCGGCCGCTGTGCCTTCTAGTTGCCAGC CATC (SEQ ID NO: 2) (containing a XhoI and a NotI restriction site) and WdV102: GGTACCATAGAGCCCACCGCATCCCCAGCATGCC (SEQ ID NO: 3) (containing a KpnI restriction site) and WdV103: GGCCGCTTTATTAATTAAGCCCTGCAGGTTGTTTAAACTTGGCGCGCCTTAT (SEQ ID. NO: 4) (contains from 5' to 3' sequentially a NotI sticky restriction site, a PacI, SbfI, PmeI and an AscI intact restriction site and a ClaI sticky restriction site) and WdV104: CGATAAGGCGCGCCAAGTTTAAACAACCTGCAGGGCTTAATTAATAAAGC (SEQ ID NO: 5) (contains from 3' to 5' sequentially a NotI sticky restriction site, a PadI, SbfI, PmeI and an AscI intact restriction site and a ClaI sticky restriction site) and WdV105: CGGGATCCAGACATGATAAGATACATTG (SEQ ID NO: 6) (containing a BamHI restriction site) and WdV106: ATAGTTTAGCGGCCGCAATGAATGCAATTGTTGTTGTTAACTTG (SEQ ID NO: 7) (containing a Notl restriction site) and WdV108: TGGCGCGCCTATAGGGAGACCCAAGCTGGCTAG (SEQ ID NO: 8) (containing an Ascl restriction site) and WdV109 : CAATCATACCGTTTAAACGAACCGCGGGCCCTCTAGAC (SEQ ID NO: 9) (containing a Pmel restriction site).

Purified plasmid DNA of the SV40 vector pSL-PL (De La Luna S. et al., Journal of General Virology 74: 535-539, 1993) was subjected to PCR using oligonucleotides WdV105 and WdV106. The resulting amplified DNA fragment comprised the SV40-polyadenylation signal flanked by a BamHI restriction site at the 5' end and a Notl restriction site at the 3' end. This SV40 polyadenylation signal fragment was digested with BamHI and Notl and the resulting 150 base pairs long DNA fragment was isolated from an agarose gel and cloned into a likewise digested pBluescript SK- plasmid (Stratagene), yielding pAM002.

Purified pEF5/FRT/5-DEST (Invitrogen) plasmid DNA was subjected to PCR using oligonucleotides WdV101 and WdV102.The resulting amplified DNA fragment comprising the bovine growth hormone (BGH) polyadenylation signal flanked by sequentially a Xhol and a Notl restriction site at the 5' end and a Kpnl restriction site at the 3' end. This BGH polyadenylation signal fragment was digested with Kpnl and Notl, and the resulting 250 base pairs long DNA fragment was isolated from an agarose gel and ligated into the likewise digested pAM002 plasmid. Transformation with this ligation mixture was performed in a methylation insensitive *E. coli* strain. This resulted in plasmid pAM003.

The two complementary oligonucleotides WdV103 and WdV104 were annealed by incubating them in a water bath that was cooling down autonomously from boiling temperature to room temperature, yielding a DNA linker containing sequentially a Notl sticky restriction site, a Pacl, Sbfl, Pmel and an Ascl intact restriction sites and a Clal sticky restriction site. This linker was ligated into the pAM003 plasmid that was digested with Notl and Clal and isolated from an agarose gel. The ligation mixture was subsequently used to transform a methylation insensitive *E. coli* strain, yielding pAM004.

Purified plasmid DNA of the SV40 vector pSL-PL was digested with Clal and BamHI. The resulting 2.6 kilo base pairs DNA fragment that contains the SV40 origin and the SV40 late region is purified from agarose and cloned into likewise digested pAM004. This resulted in the new SV40 vector plasmid pAM005.

Purified pEF5/FRT/5-DEST (Invitrogen) plasmid DNA was subjected to PCR using oligonucleotides WdV108 and WdV109. The resulting amplified DNA fragment comprises the Gateway recombination cassette including the ccdB gene and a chloramphenicol resistance gene flanked by AttR1 and AttR2 recombination sequences. These in turn are flanked by an Ascl restriction site at the 5' end and a Pmel restriction site at the 3' end. This fragment was subsequently digested with Ascl and Pmel, and the resulting 1844 base pairs long DNA fragment was isolated from an agarose gel and ligated into the likewise digested pAM005 plasmid, resulting in pAM006 (pSV*ac*-*dest*).

Correct cloning of the cassette was confirmed by sequencing. Functionality was confirmed by successful recombination of transgenes and by showing that the Chloramphenicol-resistance and the ccdB gene in the gateway cassette were functional.

### Example 2: Molecular cloning of an SV40 encoding Luciferase expression vector (SVLuc).

The expression plasmid pGL3 (Promega) was used as template for cloning of the firefly luciferase (*Photinus pyralis*) into pSVac destination vector by PCR. Two oligonucleotides were designed:
WdV-070: GGGGACAAGTTTGTACAAAAAAGCAGGCTATGGAAGACGCCAAAAACATAAAGAAAG GC (SEQ ID NO: 10) and
WdV-071: GGGGACCACTTTGTACAAGAAAGCTGGGTTTACACGGCGATCTTTCCGCCCTTC (SEQ ID NO: 11) containing respectively AttB1 and AttB2 recombination sequences.

Purified pGL3 plasmid DNA was subjected to PCR using oligonucleotides WdV070 and WdV071. The purified PCR product was subsequently recombined by a BP reaction in pDONR221 (Invitrogen) to create a luciferase entry clone, pAM007. This entry clone was used for a gateway LR reaction to recombine the firefly luciferase coding sequence into pSVac-dest (pAM006), resulting in the pSV*Luc* vector plasmid (pAM008).

### Example 3: Molecular cloning of an SV40 encoding myelin oligodendrocyte glycoprotein expression vector (SVMOG).

The coding DNA sequence for the human myelin oligodendrocyte glycoprotein (MOG, NCBI gene ID 4340) flanked by AttB1 and AttB2 recombination sites was synthesized chemically (GeneArt). The DNA fragment was subsequently recombined by a BP reaction in pDONR221 to create a MOG entry clone, pAM009. This entry clone was used for a gateway LR reaction to recombine the MOG coding sequence into pSVac-dest (pAM006), resulting in the pSV*MOG* vector plasmid (pAM010).

### Example 4: Production and purification of SVLuc and SVMOG particles.

Recombinant SV40 vector DNA encoding either the human MOG protein and firefly luciferase (pSV*MOG* and pSV*Luc*) were Notl digested to remove the bacterial backbone. The SV40 vector DNA was isolated from agarose gel and purified. Subsequently, the SV40 vector sequence was re-circularized using T4 ligase and used to transfect SuperVero cells growing in roller bottles (growth area: 850 squared centimeter; Greiner Bio One) at 20-70 percent confluence in OptiPRO™ serum free media containing L-Glutamine 200 millimolar at 37 degrees Celsius and 5 percent carbon dioxide. SuperVero is a genetically engineered packaging cell line that was generated by the introduction of polyomaviral SV40 large T antigen into Vero cells.

SuperVero cells support the replication of replication-deficient recombinant SV40 (rSV40) vectors as described in detail in patent application WO/2010/122094.

Three and six days post transfection, the culture medium containing the vector particles was collected and replaced by fresh media. The pooled culture media containing SV*MOG* or SV*Luc* were purified using a dual membrane filter (1/0.5 micrometer Polysep II filter, Millipore) and 10 times concentrated by ultrafiltration/diafiltration (UFDF), a tangential flow filtration performed using a 300 kilodalton molecular weight cut-off membrane (Pall Corporation). Finally, the vector material was filtered through a 0.45 micrometer Acrodisc filter (Pall Corporation), aliquoted and stored at 4 degrees Celsius.

To further increase the amount of vector particles pooled the vector was used for (at least) two subsequent transduction rounds. In each transduction round, SuperVero cells were transduced with 400 SV*MOG* or SV*Luc* vector genomes per cell. Three and six days post transduction, the rSV40 particle-containing culture medium was collected and replaced by fresh media. After each amplification round, the pooled culture medium was purified and concentrated as described above.

A third transduction round was performed on at least 40 roller bottles (growth area: 850 squared centimeter) containing 20-70 percent confluent SuperVero cells transduced with 400 SV*MOG* or SV*Luc* vector genomes per cell. Again, at three and six days post transduction the rSV40 vector particles containing culture medium was collected and replaced by fresh media. The collected culture media results in a final batch volume of at least 20 liter.

To remove unwanted nucleic acids (mainly in the form of host cell DNA), Benzonase was added (10 Units/milliliter, VWR International) in the presence of 2 millimolar MgCl₂ and incubated for 1-4 hours at room temperature. The material was subsequently clarified using a dual membrane filter (1/0.5 micrometer Polysep II filter) in order to remove the cell debris. After the clarification, the material was concentrated 10-60 fold using an UFDF step, followed by filtration through a 0.45 micrometer Millipack membrane (Millipore).

Remaining impurities were then removed from the vector material by a group separation chromatography using an AKTA explorer, 2.5 liter, 40 centimeters Sepharose 6 fast flow resin column (GE Healthcare Life Science). Ultraviolet (UV) absorbance at 260 and 280 nanometers was used for detection of vector particles and fractions containing the vector peak were mixed and stored overnight at 4 degrees Celsius. The column eluate pool containing SV*MOG* or SV*Luc* vector particles was concentrated to approximately 15 milliliter and the buffer was exchanged 10-fold by UFDF. Sucrose (5 percent volume/volume) and tween-80 (0.005 percent volume/volume) excipients were added and the final concentrated bulk product was sterile filtered (0.8/0.2 micrometer Acrodisc PF filter, Pall Corporation), aliquoted in glass vials and stored at 4 degrees Celsius until use.

### Example 5: Amelioration of clinical EAE symptoms by prophylactic and therapeutic SVMOG administration in marmoset (Callithrix jacchus).

A proof of concept study was conducted in which six bone marrow-chimeric twins were treated in a prophylactic setting: of each twin one sibling received one intravenous administration of the MOG encoding vector (SV*MOG*) and the other the control vector encoding luciferase (SV*Luc*). The animals were administered with 1 x10¹¹ vector genomes. Subsequently, EAE was induced in marmosets using peptide, consisting of amino acid 34 to 56 of MOG (MOG34-56) in incomplete Freund's adjuvant (IFA; Difco Laboratories; Detroit MI)(Billiau A. and Matthys P., Journal of Leukocyte Biology 70: 849-860,2001).

In parallel, EAE was induced in six bone marrow-chimeric twins using MOG34-56 in incomplete Freund's adjuvant. Subsequently, the animals were treated in a therapeutic setting: of each twin one sibling received one intravenous administration of SV*MOG* and the other sibling was administered with the control vector SV*Luc*. The animals were administered with 1 x 10¹¹ vector genomes.

The resulting disease course was characterized by quantifying lesions with inflammation and demyelination within the central nervous system (CNS) white and grey matter. This new and highly refined model shares essential clinical, radiological and pathological similarities with human MS, and has been described in detail in previous publications (Bert A. 't Hart et al., Trends in Molecular Medicine 17:119-25, 2011; Kap YS et al., Journal of Neuroimmune Pharmacology 5:220-30, 2010). The severity of EAE was scored daily by two independent observers on a quantitative scale (Table 1).

**Table 1. Integrated discomfort scoring table**

| **Discomfod Score** | **Clinical signs** | **Monitoring** | **Maximal duration** |
|---|---|---|---|
| 0 | Asymptomatic No general discomfort signs | Daily | End of experiment |
| 0.5 | Reduced alertness, loss of appetite, altered walking pattern without ataxia | Daily | 20 weeks |
| 1 | lethargy and/or weight loss less than 15% from start weight | Daily | 10 weeks |
| 2 | Ataxia (= reduced capacity to keep Balance), visual disturbance, optic neuritis | Daily | 6 weeks |
| 2.5 | **Incomplete paralysis:** | Daily | < 7 days |
| | para- or monoparesis and/or sensory loss and/or brain stem syndrome | | |
| 3.0 | **Complete paralysis** | Daily | < 3 days |
| | hind part of the body one- (hemiplegia) or two-sided (paraplegia) | | |
| 4.0^{#} | **Complete paralysis** | Daily | < 18 hours |
| | all four limbs quadriplegia | | |
| 5.0^{#} | Lethargy (no reaction to external stimuli); incapacity to eat or drink without help, self mutilation, blindness more than two days, untreatable pain | Daily | < 1 hour |

| | | | |
|---|---|---|---|
| #: EAE scores 4 and 5 are normally not observed as they are beyond the ethical end-point. However, rarely the disease progresses very fast. Therefore, expression of scores 4 and 5 can be avoided. | | | |

It was found that prophylactic and therapeutic treatment of marmosets with SV*MOG* significantly ameliorates the development of EAE symptoms. It was also found that there was no adverse immune response against the antigen MOG during the time of observation when the antigen was expressed under transcriptional control of the SV40 early and late promoter, whereas a vast immune response was observed against MOG in animals immunized with MOG34-56 in incomplete Freund's adjuvant.

### Example 6: Amelioration of disease symptoms of other neurological degenerative diseases by prophylactic and therapeutic administration of a replication-deficient SV40 vector encoding a major auto-antigen in mouse (Mus musculus).

Recombinant SV40 vector DNA encoding one or more auto-antigens or parts thereof selected from the group consisting of neurofilament light (NFL) chain, alpha-actinin, glutamate receptor type 3 (GluR3), endothelial cell growth factor, acetylcholine receptor, hypocretin/orexin, aquaporin 4, and other auto-antigens identified using the confirmatory methods described herein above or using other immunological methods known to those skilled in the art, may be constructed after obtaining the DNA encoding the auto-antigens or parts thereof as described in example 3 using chemical synthesis (GeneArt). The DNA fragment may be subsequently recombined into pSVac-dest (pAM006) by Gateway Technology (Invitrogen) resulting in a pSVac expression plasmid DNA used to produce and purify SVac particles encoding said auto- as described in example 4. These vector particles may be intravenously administered in animal model relevant to human neurological degenerative diseases such as Alzheimer's dementia, Amyotrophic lateral sclerosis, Bipolar disorder, Depression, Epilepsy, Huntington disease, Lyme disease, Multiple system atrophy, Myasthenia gravis, Narcolepsy, Neuromyelitis optica, Parkinson disease and Schizophrenia. It is described herein that the animals may be treated in a prophylactic setting, whereasit is also described that the animals may be treated in a therapeutic setting as described in example 5. The therapeutic effect of SVac administration is then evaluated for its ability to ameliorate the development of the neurological degenerative disease symptoms. Moreover, the adverse immune response against the auto-antigen may be monitored over time. It will be found that prophylactic and therapeutic treatment of animals with a SVac vector encoding a major auto-antigen involved in the autoimmune neural tissue destruction significantly ameliorates the development of disease symptoms. It will also be found that there is no adverse immune response against the auto-antigen during the time of observation when the auto-antigen is expressed under transcriptional control of the SV40 early and late promoter.

### Example 7: Amelioration of disease symptoms of metabolic degenerative diseases by prophylactic and therapeutic administration of a replication-deficient SV40 vector encoding a major auto-antigen in mouse (Mus musculus)

Recombinant SV40 vector DNA encoding one or more auto-antigens or parts thereof selected from the group consisting of proinsulin, glutamic acid decarboxylase 65, thyroglobulin, Golgi, SNAP receptor complex member 1 (GOSR1), and other auto-antigens identified using the confirmatory methods described herein above or using other immunological methods known to those skilled in the art, may be constructed after obtaining the DNA encoding the auto-antigens or parts thereof as described in example 3 using chemical synthesis (GeneArt). The DNA fragment may subsequently be recombined into pSVac-dest (pAM006) by Gateway Technology (Invitrogen) resulting in a pSVac expression plasmid DNA used to produce and purify SVac particles encoding said auto-antigens as described in example 4. These vector particles may be intravenously administered in animal model relevant to human metabolic degenerative diseases such as Type 1 diabetes, Type 2 diabetes, Hashimoto's thyroiditis and Obesitas. It is described herein that the animals may be treated in a prophylactic setting, whereas it is also described that the animals may be treated in a therapeutic setting as described in example 5. The therapeutic effect of SVac administration may be evaluated for its ability to ameliorate the development of the metabolic degenerative disease symptoms. Moreover, the adverse immune response against the auto-antigen may be monitored over time. It will be found that prophylactic and therapeutic treatment of animals with a SVac vector encoding a major auto-antigen involved in the autoimmune tissue destruction significantly ameliorates the development of disease symptoms. It may also be found that there is no adverse immune response against the auto-antigen during the time of observation when the auto-antigen was expressed under transcriptional control of the SV40 early and late promoter.

### Example 8: Amelioration of disease symptoms of ophthalmological degenerative diseases by prophylactic and therapeutic administration of a replication-deficient SV40 vector encoding a major auto-antigen in mouse (Mus musculus).

Recombinant SV40 vector DNA encoding one or more auto-antigens or parts thereof selected from the group consisting of arrestin, type I collagen, interphotoreceptor retinoid-binding protein, and other auto-antigens identified using the confirmatory methods described herein above or using other immunological methods known to those skilled in the art, may be constructed after obtaining the DNA encoding the auto-antigens or parts thereof as described in example 3 using chemical synthesis (GeneArt). The DNA fragment may be subsequently recombined into pSVac-dest (pAM006) by Gateway Technology (Invitrogen) resulting in a pSVac expression plasmid DNA used to produce and purify SVac particles encoding said auto-antigens as described in example 4. These vector particles may be intravenously administered in animal model relevant to human ophthalmological degenerative diseases such as Autoimmune retinopathy, Age-related macular degeneration, Glaucoma, Uveitis, Retinitis pigmentosa, Leber's congenital amaurosis, Stargardt macular dystrophy, Achromatopsia, Retinoschisis and Vitelliform macular dystrophy. It is described herein that the animals may be treated in a prophylactic setting, whereas it is also described herein that the animals may treated in a therapeutic setting as described in example 5. The therapeutic effect of SVac administration may be evaluated for its ability to ameliorate the development of the ophthalmological degenerative disease symptoms. Moreover, the adverse immune response against the auto-antigen may be monitored over time. It will be found that prophylactic and therapeutic treatment of animals with a SVac vector encoding a major auto-antigen involved in the autoimmune retinal tissue destruction significantly ameliorates the development of disease symptoms. It will also be found that there is no adverse immune response against the auto-antigen during the time of observation when the auto-antigen was expressed under transcriptional control of the SV40 early and late promoter.

### Example 9: Amelioration of disease symptoms of gastro-enteric degenerative diseases by prophylactic and therapeutic administration of a replication-deficient SV40 vector encoding a major auto-antigen in mouse (Mus musculus).

Recombinant SV40 vector DNA encoding one or more auto-antigens or parts thereof selected from the group consisting of transglutaminase, forminotransferase cyclodeaminase and other auto-antigens identified using the confirmatory methods described herein above or using other immunological methods known to those skilled in the art, may be constructed after obtaining the DNA encoding the auto-antigens or parts thereof as described in example 3 using chemical synthesis (GeneArt). The DNA fragment may subsequently be recombined into pSVac-dest (pAM006) by Gateway Technology (Invitrogen) resulting in a pSVac expression plasmid DNA used to produce and purify SVac particles encoding said auto-antigens as described in example 4. These vector particles may be intravenously administered in animal model relevant to human gastro-enteric degenerative diseases such as Celiac disease, Autoimmune hepatitis and inflammatory bowel diseases including Crohn's disease and Ulcerative colitis. It is described herein that the animals may be treated in a prophylactic setting, whereas it is also described that the animals may be treated in a therapeutic setting as described in example 5. The therapeutic effect of SVac administration may be evaluated for its ability to ameliorate the development of the gastro-enteric degenerative disease symptoms. Moreover, the adverse immune response against the auto-antigen may be monitored over time. It will be found that prophylactic and therapeutic treatment of animals with an SVac vector encoding a major auto-antigen involved in the autoimmune tissue destruction significantly ameliorates the development of disease symptoms. It will also be found that there is no adverse immune response against the auto-antigen during the time of observation when the auto-antigen was expressed under transcriptional control of the SV40 early and late promoter.

### Example 10: Amelioration of disease symptoms of dermatological degenerative diseases by prophylactic and therapeutic administration of a replication-deficient SV40 vector encoding a major auto-antigen in mouse (Mus musculus).

Recombinant SV40 vector DNA encoding one or more auto-antigens or parts thereof selected from the group consisting of keratin, alpha-fodrin, and other auto-antigens identified using the confirmatory methods described herein above or using other immunological methods known to those skilled in the art, may be constructed after obtaining the DNA encoding the auto-antigens or parts thereof as described in example 3 using chemical synthesis (GeneArt). The DNA fragment may subsequently be recombined into pSVac-dest (pAM006) by Gateway Technology (Invitrogen) resulting in a pSVac expression plasmid DNA used to produce and purify SVac particles encoding said auto-antigens as described in example 4. These vector particles may be intravenously administered in animal model relevant to human dermatological degenerative diseases such as Psoriasis, Scleroderma, Sjögren's syndrome and Vitiligo. It is described herein that the animals may be treated in a prophylactic setting, whereas it is also described that the animals may be treated in a therapeutic setting as described in example 5. The therapeutic effect of SVac administration may be evaluated for its ability to ameliorate the development of the dermatological degenerative disease symptoms. Moreover, the adverse immune response against the auto-antigen may be monitored over time. It will be found that prophylactic and therapeutic treatment of animals with an SVac vector encoding a major auto-antigen involved in the autoimmune skin destruction significantly ameliorates the development of disease symptoms. It will also be found that there is no adverse immune response against the auto-antigen during the time of observation when the auto-antigen was expressed under transcriptional control of the SV40 early and late promoter.

### Example 11: Amelioration of disease symptoms of cardio-vascular degenerative diseases by prophylactic and therapeutic administration of a replication-deficient SV40 vector encoding a major auto-antigen in mouse (Mus musculus).

Recombinant SV40 vector DNA encoding one or more auto-antigens or parts thereof selected from the group consisting of elastin, beta-2-glycoprotein I, and other auto-antigens identified using the confirmatory methods described herein above or using other immunological methods known to those skilled in the art, may be constructed after obtaining the DNA encoding the auto-antigens or parts thereof as described in example 3 using chemical synthesis (GeneArt). The DNA fragment may subsequently be recombined into pSVac-dest (pAM006) by Gateway Technology (Invitrogen) resulting in a pSVac expression plasmid DNA used to produce and purify SVac particles encoding said auto-antigens as described in example 4. These vector particles may be intravenously administered in animal model relevant to human cardio-vascular degenerative diseases such as Atherosclerosis, Cardiomyopathy and Coxsackie myocarditis. It is described herein that the animals may be treated in a prophylactic setting, whereas it is also described that the animals may be treated in a therapeutic setting as described in example 5. The therapeutic effect of SVac administration may be evaluated for its ability to ameliorate the development of the cardio-vascular degenerative disease symptoms. Moreover, the adverse immune response against the auto-antigen may be monitored over time. It will be found that prophylactic and therapeutic treatment of animals with an SVac vector encoding a major auto-antigen involved in the autoimmune endothelial tissue destruction significantly ameliorates the development of disease symptoms. It will also be found that there is no adverse immune response against the auto-antigen during the time of observation when the auto-antigen was expressed under transcriptional control of the SV40 early and late promoter.

### Example 12: Amelioration of disease symptoms of orthopedic degenerative diseases by prophylactic and therapeutic administration of a replication-deficient SV40 vector encoding a major auto-antigen in mouse (Mus musculus).

Recombinant SV40 vector DNA encoding one or more auto-antigens or parts thereof selected from the group consisting of RNA-induced silencing complex (RISC) components, type II collagen, and other auto-antigens identified using the confirmatory methods described herein above or using other immunological methods known to those skilled in the art, may be constructed after obtaining the DNA encoding the auto-antigens or parts thereof as described in example 3 using chemical synthesis (GeneArt). The DNA fragment may subsequently be recombined into pSVac-dest (pAM006) by Gateway Technology (Invitrogen) resulting in a pSVac expression plasmid DNA used to produce and purify SVac particles encoding said auto-antigens as described in example 4. These vector particles may be intravenously administered in animal model relevant to human orthopedic degenerative diseases such as Rheumatoid arthritis, Rheumatic fever and Lupus erythematosus. It is described herein that the animals may be treated in a prophylactic setting, whereas it is also described that the animals may be treated in a therapeutic setting as described in example 5. The therapeutic effect of SVac administration may be evaluated for its ability to ameliorate the development of the orthopedic degenerative disease symptoms. Moreover, the adverse immune response against the auto-antigen may be monitored over time. It will be found that prophylactic and therapeutic treatment of animals with an SVac vector encoding a major auto-antigen involved in the autoimmune tissue destruction significantly ameliorates the development of disease symptoms. It will also be found that there is no adverse immune response against the auto-antigen during the time of observation when the auto-antigen was expressed under transcriptional control of the SV40 early and late promoter.

### Example 13: Amelioration of disease symptoms of muscular degenerative diseases by prophylactic and therapeutic administration of a replication-deficient SV40 vector encoding a major auto-antigen in mouse (Mus musculus).

Recombinant SV40 vector DNA encoding one or more auto-antigens or parts thereof such as histidyl-tRNA-synthetase, and other auto-antigens identified using the confirmatory methods described herein above or using other immunological methods known to those skilled in the art, may be constructed after obtaining the DNA encoding the auto-antigens or parts thereof as described in example 3 using chemical synthesis (GeneArt). The DNA fragment may subsequently be recombined into pSVac-dest (pAM006) by Gateway Technology (Invitrogen) resulting in a pSVac expression plasmid DNA used to produce and purify SVac particles encoding said auto-antigens as described in example 4. These vector particles may be intravenously administered in animal model relevant to human muscular degenerative diseases such as Polymyositis, Dermatomyositis, inclusion body myositis, Duchenne muscular dystrophy, Becker muscular dystrophy, Miyoshi myopathy, Limb-girdle muscular dystrophy, Congenital muscular dystrophy, Distal muscular dystrophy, Emery-Dreyfuss muscular dystrophy, Fascio-scapulohumeral muscular dystrophy, Myotonic muscular dystrophy and Oculopharyngeal muscular dystrophy. It is described herein that the animals may be treated in a prophylactic setting, It is also described that the animals may be treated in a therapeutic setting as described in example 5. The therapeutic effect of SVac administration may be evaluated for its ability to ameliorate the development of the muscular degenerative disease symptoms. Moreover, the adverse immune response against the auto-antigen may be monitored over time. It will be found that prophylactic and therapeutic treatment of animals with an SVac vector encoding a major auto-antigen involved in the autoimmune muscle destruction significantly ameliorates the development of disease symptoms. It will also be found that there is no adverse immune response against the auto-antigen during the time of observation when the auto-antigen was expressed under transcriptional control of the SV40 early and late promoter.

### Example 14: Amelioration of disease symptoms of pulmonary degenerative diseases by prophylactic and therapeutic administration of a replication-deficient SV40 vector encoding a major auto-antigen in mouse (Mus musculus).

Recombinant SV40 vector DNA encoding one or more auto-antigens or parts thereof selected from the group consisting of elastin, beta-2-glycoprotein I, and other auto-antigens identified using the confirmatory methods described herein above or using other immunological methods known to those skilled in the art, may be constructed after obtaining the DNA encoding the auto-antigens or parts thereof as described in example 3 using chemical synthesis (GeneArt). The DNA fragment may subsequently be recombined into pSVac-dest (pAM006) by Gateway Technology (Invitrogen) resulting in a pSVac expression plasmid DNA used to produce and purify SVac particles encoding said auto-antigens as described in example 4. These vector particles may be intravenously administered in animal model relevant to human pulmonary degenerative diseases such as Chronic obstructive pulmonary disease and Asthma. It is described herein that the animals may be treated in a prophylactic setting, whereas it is also described that the animals may be treated in a therapeutic setting as described in example 5. The therapeutic effect of SVac administration may be evaluated for its ability to ameliorate the development of the pulmunary degenerative disease symptoms. Moreover, the adverse immune response against the auto-antigen may be monitored over time. It will be found that prophylactic and therapeutic treatment of animals with an SVac vector encoding a major auto-antigen involved in the autoimmune lung destruction significantly ameliorates the development of disease symptoms. It will also be found that there is no adverse immune response against the auto-antigen during the time of observation when the auto-antigen was expressed under transcriptional control of the SV40 early and late promoter.

### Example 15: Testing the ability of viral promoters to induce RNA interference.

The VP35 open reading frame from Ebola virus strain Zaire was cloned into the mammalian expression vector pEF5-V5-DEST containing the human EF1-alpha promoter using GATEWAY technology (Invitrogen) as previously described (Haasnoot J. et al., Plos Pathogens 3: e86, 2007). Human embryonic kidney (HEK293T) cells were grown as a monolayer in DMEM (Gibco) supplemented with 10 percent of fetal bovine serum (FBS) (Gibco) at 37 degrees Celsius and 5 percent of CO₂. One day before transfection, cells were trypsinized, resuspended in DMEM, and seeded in 24-well plates at a density of 1.5 × 10⁵ cells per well. At the time of transfection, the cells were 60 to 70 percent confluent. The transfection was performed in quadruplicates using Lipofectamine 2000 (Invitrogen) according to the instructions of the manufacturer. For the luciferase and renilla assay, cells were co-transfected with increasing amounts (0, 10, 50, 100 nanograms) of expression construct encoding the RNA silencing suppressor VP35 and either 2 nanograms of expression plasmid encoding the *Renilla* luciferase under the control of CMV immediate early promoter (pRL-CMV), 100 nanograms of expression plasmid encoding the firefly luciferase under the control of SV40 early promoter (pGL3) or 100 nanograms of expression plasmid encoding the firefly luciferase under transcriptional control of the full-length SV40 early and late promoter (SV*Luc,* SEQ ID NO: 12). Lysates from cultured cells were prepared 2 days after transfection as follows: Cell culture medium was removed and cells were rinsed with 100 microliters of phosphate buffered saline (PBS). Cells were then lysed in 100 microliters of 1x Passive Lysis Buffer (PLB, Promega) by shaking for 30 minutes at room temperature. Firefly or *Renilla* luciferase expression was measured in 10 microliters of supernatant with the dual luciferase reporter assay system (Promega).

HEK293T cells transfected with plasmids where reporter gene expression was driven by the CMV immediate early promoter or the SV40 early promoter (pRL-CMV and pGL3), in the presence of an expression plasmid encoding a viral suppressor of RNA interference; VP35, accumulate high amounts of reporter protein in a dose-dependent manner (Figures 1A and B respectively). HEK293T cells transfected with plasmids where reporter gene expression was driven by the full-length SV40 early and late promoter (SV*Luc*, SEQ ID NO: 12) accumulated high amounts of reporter gene independently of the presence or absence of VP35 (Figure 1 C). From these experiments it may be concluded that the CMV immediate early promoter and the SV40 early promoter induce RNA interference. The full length SV40 early and late promoter does not induce RNA interference in mammalian cells.

## Claims

1. A replication-defective SV40 vector particle comprising a nucleic acid sequence encoding an auto-antigen under the transcriptional control of a full-length SV40 early and late promoter according to SEQ ID NO: 12, for use in a method of treatment by inducing an immune tolerance towards the auto-antigen in a host organism.

2. A replication-defective SV40 vector particle for use according to claim 1 wherein the auto-antigen is selected from the group consisting of alpha-fodrin, arrestin, neurofilament light chain, alpha-actinin, beta-2 glycoprotein I, cochlin, beta-tectorin, elastin, the glutamate receptor type 3, forminotransferase cyclodeaminase, tissue transglutaminase, type I collagen, type II collagen, type IV collagen, interphotoreceptor retinoid-binding protein, thyroglobulin, platelet-specific glycoproteins IIb/IIIa, Ib/IX, Ia/IIa and IV, endothelial cell growth factor, myosin-binding C protein, histidyl-tRNA-synthetase, the acetylcholine receptor, hypocretin-1/orexin, aquaporin 4, RNA-induced silencing complex (RISC) components, myelin oligodendrocyte glycoprotein, myelin basic protein, Golgi SNAP receptor complex member 1, keratin, proinsulin and glutamic acid decarboxylase 65, Elastin, the glutamate receptor type 3 (GluR3), interphotoreceptor retinoid-binding protein or an antigenic part thereof.

3. A replication-defective SV40 vector particle for use according to claims 1 or 2 wherein the host has a disease selected from the group consisting of neurological diseases consisting of Alzheimer's dementia, Amyotrophic lateral sclerosis, Bipolar disorder, Depression, Epilepsy, Huntington disease, Lyme disease, Multiple system atrophy, Multiple sclerosis, Myasthenia gravis, Narcolepsy, Neuromyelitis optica, Parkinson disease, Schizophrenia, Type 1 diabetes, Type 2 diabetes, Hashimoto's thyroiditis, obesity, ophthalmological diseases consisting of autoimmune retinopathy, Age-related macular degeneration, Glaucoma, Uveitis, Retinitis pigmentosa, Leber's congenital amaurosis, Stargardt macular dystrophy, Achromatopsia, Retinoschisis, Vitelliform macular dystrophy, gastro-enteric diseases consisting of Celiac disease and inflammatory bowel diseases including Crohn's disease, Ulcerative colitis, Psoriasis, Scleroderma, Sjögren's syndrome, Vitiligo, cardio-vascular diseases, Atherosclerosis, Cardiomyopathy, Coxsackie myocarditis, orthopedic diseases, Rheumatoid arthritis, Rheumatic fever, Lupus erythematosus, muscular diseases, Polymyositis, Dermatomyositis, inclusion body myositis, Duchenne muscular dystrophy, Becker muscular dystrophy, Miyoshi myopathy, Limb-girdle muscular dystrophy, Congenital muscular dystrophy, Distal muscular dystrophy, Emery-Dreyfuss muscular dystrophy, Fascio-scapulohumeral muscular dystrophy, Myotonic muscular dystrophy, Oculopharyngeal muscular dystrophy, pulmonary diseases, Chronic obstructive pulmonary disease and asthma.

4. A replication-defective SV40 vector particle for use according to any one of claims 1 - 3 wherein the host is human.

5. A replication-defective SV40 vector particle for use according to any one of claims 1 - 4 wherein the use includes administering to the host a replication-defective SV40 vector particle comprising a nucleic acid sequence encoding an auto-antigen under the transcriptional control of a full-length SV40 early and late promoter according to SEQ ID NO:12.

6. A replication-defective SV40 vector particle for use according to claim 5 wherein the vector particle is administered intravenously.

## Patentansprüche

1. Replikationsdefektes SV40-Vektorpartikel, umfassend eine Nukleinsäuresequenz, die ein Autoantigen unter der Transkriptionskontrolle eines Volllängen-SV40 early und late Promotors gemäß SEQ ID NO: 12 codiert, zur Verwendung bei einem Behandlungsverfahren durch Induzieren einer Immuntoleranz gegenüber dem Autoantigen in einem Wirtsorganismus.

2. Replikationsdefektes SV40-Vektorpartikel zur Verwendung nach Anspruch 1, wobei das Autoantigen ausgewählt ist aus der Gruppe bestehend aus Alpha-Fodrin, Arrestin, Neurofilament-Leichtekette, Alpha-Actinin, Beta-2-Glycoprotein I, Cochlin, Beta-Tectorin, Elastin, dem Glutamatrezeptor Typ 3, Forminotransferase-Cyclodeaminase, Gewebetransglutaminase, Kollagen Typ I, Kollagen Typ II, Kollagen Typ IV, Interphotorezeptor-Retinoid-Bindungsprotein, Thyroglobulin, plättchenspezifischen Glycoproteinen IIb/IIIa, Ib/IX, Ia/IIa und IV, endothelialem Zellwachstumsfaktor, Myosin-bindenden C-Protein, Histidyl-tRNA-Synthetase, dem Acetylcholinrezeptor, Hypocretin-1/Orexin, Aquaporin 4, Komponenten des RNA-induzierten Silencing-Komplex (RISC), Myelin-Oligodendrozyten-Glycoprotein, Myelin-Basisprotein, Golgi-SNAP-Rezeptor-Komplexmitglied 1, Keratin, Proinsulin und Glutaminsäure-Decarboxylase 65, Elastin, dem Glutamatrezeptor Typ 3 (GluR3), Interphotorezeptor-Retinoid-Bindungsprotein oder einem antigenen Teil davon.

3. Replikationsdefektes SV40-Vektorpartikel zur Verwendung nach den Ansprüchen 1 oder 2, wobei der Wirt eine Krankheit hat, die ausgewählt ist aus der Gruppe bestehend aus neurologischen Erkrankungen, bestehend aus Alzheimer-Demenz, amyotropher Lateralsklerose, bipolarer Störung, Depression, Epilepsie, Chorea Huntington, Lyme-Borreliose, multipler Systematrophie, multipler Sklerose, Myasthenia gravis, Narkolepsie, Neuromyelitis optica, Parkinson-Krankheit, Schizophrenie, Diabetes Typ 1, Diabetes Typ 2, Hashimoto-Thyreoiditis, Adipositas, Augenerkrankungen, bestehend aus Autoimmun-Retinopathie, altersbedingter Makuladegeneration, Glaukom, Uveitis, Retinitis pigmentosa, Leberscher kongenitaler Amaurose, Stargardt-Makuladystrophie, Achromatopsie, Retinoschisis, vitelliformer Makuladystrophie, Magen-Darm-Erkrankungen, bestehend aus Zöliakie und entzündlichen Darmerkrankungen, einschließlich Morbus Crohn, Colitis ulcerosa, Psoriasis, Sklerodermie, Sjögren-Syndrom, Vitiligo, Herz-Kreislauf-Erkrankungen, Atherosklerose, Kardiomyopathie, Coxsackie-Myocarditis, orthopädischen Erkrankungen, rheumatoider Arthritis, rheumatischem Fieber, Lupus erythematodes, Muskelerkrankungen, Polymyositis, Dermatomyositis, Einschlusskörper-Myositis, Duchenne-Muskeldystrophie, Becker-Muskeldystrophie, Miyoshi-Myopathie, Gliedergürtel-Muskeldysthrophie, kongenitaler Muskeldystrophie, distaler Muskeldystrophie, Emery-Dreyfuss-Muskeldystrophie, fascioscapulohumeraler Muskeldystrophie, myotonischer Muskeldystrophie, okulopharyngealer Muskeldystrophie, Lungenerkrankungen, chronisch obstruktiver Lungenerkrankung und Asthma.

4. Replikationsdefektes SV40-Vektorpartikel zur Verwendung nach einem der Ansprüche 1 bis 3, wobei der Wirt ein Mensch ist.

5. Replikationsdefektes SV40-Vektorpartikel zur Verwendung nach einem der Ansprüche 1 bis 4, wobei die Verwendung die Verabreichung eines replikationsdefekten SV40-Vektorpartikels, das eine Nukleinsäuresequenz umfasst, die ein Autoantigen unter der Transkriptionskontrolle eines Volllängen-SV40 early und late Promotors gemäß SEQ ID NO: 12 codiert, an den Wirt umfasst.

6. Replikationsdefektes SV40-Vektorpartikel zur Verwendung nach Anspruch 5, wobei das Vektorpartikel intravenös verabreicht wird.

## Revendications

1. Particule de vecteur de SV40 déficient en réplication comprenant une séquence d'acide nucléique codant pour un autoantigène sous le contrôle transcriptionnel d'un promoteur précoce et tardif de SV40 de pleine longueur selon SEQ ID NO: 12, pour utilisation dans un procédé de traitement par induction d'une tolérance immunitaire vis-à-vis de l'autoantigène dans un organisme hôte.

2. Particule de vecteur SV40 déficient en réplication pour utilisation selon la revendication 1, dans laquelle l'autoantigène est choisi dans le groupe constitué des alpha-fodrine, arrestine, chaîne légère de neurofilament, alpha-actinine, glycoprotéine bêta-2 I, cochline, bêta-tectorine, élastine, le récepteur de glutamate de type 3, forminotransférase cyclodésaminase, transglutaminase tissulaire, collagène de type I, collagène de type II, collagène de type IV, protéine de liaison de rétinoïde interphotorécepteur, thyroglobuline, glycoprotéines spécifiques des plaquettes IIb/IIIa, Ib/IX, Ia/IIa et IV, facteur de croissance des cellules endothéliales, protéine C de liaison de myosine, histidyl-ARNt-synthétase, récepteur d'acétylcholine, hypocrétine-1/orexine, aquaporine 4, composants du complexe de silençage induit par ARN (RISC), glycoprotéine d'oligodendrocyte de myéline, protéine basique de myéline, membre 1 du complexe de récepteur SNAP de Golgi, kératine, pro-insuline et acide glutamique décarboxylase 65, élastine, récepteur de glutamate de type 3 (GluR3), protéine de liaison de rétinoïde interphotorécepteur ou une partie antigénique de celle-ci.

3. Particule de vecteur SV40 déficiente en réplication pour utilisation selon les revendications 1 ou 2, l'hôte ayant une maladie choisie dans le groupe constitué de maladies neurologiques constituées de la démence d'Alzheimer, la sclérose latérale amyotrophique, un trouble bipolaire, la dépression, l'épilepsie, la maladie de Huntington, la maladie de Lyme, l'atrophie de systèmes multiples, la sclérose en plaques, la myasthénie grave, la narcolepsie, la neuromyélite optique, la maladie de Parkinson, la schizophrénie, le diabète de type 1, le diabète de type 2, la thyroïdite de Hashimoto, l'obésité, des maladies ophtalmologiques constituées de la rétinopathie auto-immune, la dégénérescence maculaire liée à l'âge, le glaucome, l'uvéite, la rétinite pigmentaire, l'amaurose congénitale de Leber, la dystrophie maculaire de Stargardt, l'achromatopsie, le rétinoschisis, la dystrophie maculaire vitelliforme, des maladies gastro-entériques constituées de la maladie coeliaque et d'affections abdominales inflammatoires comprenant la maladie de Crohn, la recto-colite hémorragique, le psoriasis, la sclérodermie, le syndrome de Sjögren, le vitiligo, des maladies cardiovasculaires, l'athérosclérose, une cardiomyopathie, la myocardite de Coxsackie, des maladies orthopédiques, la polyarthrite rhumatoïde, le rhumatisme articulaire aigu, le lupus érythémateux, des maladies musculaires, la polymyosite, la dermatomyosite, la myosite à corps d'inclusion, la dystrophie musculaire de Duchenne, la dystrophie musculaire de Becker, la myopathie de Miyoshi, la myopathie des ceintures, la dystrophie musculaire congénitale, la dystrophie musculaire distale, la dystrophie musculaire d'Emery-Dreyfuss, la dystrophie musculaire fascio-scapulo-humérale, la dystrophie musculaire myotonique, la dystrophie musculaire oculo-pharyngée, des maladies pulmonaires, la bronchopneumopathie chronique obstructive et l'asthme.

4. Particule de vecteur SV40 déficient en réplication pour utilisation selon l'une quelconque des revendications 1 à 3, l'hôte étant humain.

5. Particule de vecteur SV40 déficient en réplication pour utilisation selon l'une quelconque des revendications 1 à 4, l'utilisation comprenant l'administration à l'hôte d'une particule de vecteur SV40 déficient en réplication comprenant une séquence d'acide nucléique codant pour un autoantigène sous le contrôle transcriptionnel d'un promoteur précoce et tardif de SV40 de pleine longueur selon SEQ ID NO: 12.

6. Particule de vecteur SV40 déficient en réplication pour utilisation selon la revendication 5, la particule de vecteur étant administrée par voie intraveineuse.


### SEQUENCE LISTING

<110> Amarna Holding BV
<120> Method for restoring immune tolerance in vivo
<130> 253 WO
<160> 12
<170> PatentIn version 3.5
<210> 1
   <211> 535
   <212> DNA
   <213> Homo sapiens
<400> 1
   tgatatctgc agaattccac cacactggac tagtggatcc gagctcggta ccaagcttac     60
   ctagccagct tgggtctccc tataggcgcg ccagttcgcg atgctgatca cgtagatctt    120
   gcatcgataa gctttttgca aaagcctagg cctccaaaaa agcctcctca ctacttctgg    180
   aatagctcag aggccgaggc ggcctcggcc tctgcataaa taaaaaaaat tagtcagcca    240
   tggggcggag aatgggcgga actgggcgga gttaggggcg ggatgggcgg agttaggggc    300
   gggactatgg ttgctgacta attgagatgc atgctttgca tacttctgcc tgctggggag    360
   cctggggact ttccacacct ggttgctgac taattgagat gcatgctttg catacttctg    420
   cctgctgggg agcctgggga ctttccacac cctaactgac acacattcca cagctggttc    480
   tttccgcctc agaaggtacc taaccaagtt cctctttcag aggttatttc aggcc         535
<210> 2
   <211> 43
   <212> DNA
   <213> Homo sapiens
<400> 2
   ccgctcgagt tgcggccgct gtgccttcta gttgccagcc atc 43
<210> 3
   <211> 34
   <212> DNA
   <213> Homo sapiens
<400> 3
   ggtaccatag agcccaccgc atccccagca tgcc 34
<210> 4
   <211> 52
   <212> DNA
   <213> Homo sapiens
<400> 4
   ggccgcttta ttaattaagc cctgcaggtt gtttaaactt ggcgcgcctt at 52
<210> 5
   <211> 50
   <212> DNA
   <213> Homo sapiens
<400> 5
   cgataaggcg cgccaagttt aaacaacctg cagggcttaa ttaataaagc 50
<210> 6
   <211> 28
   <212> DNA
   <213> Homo sapiens
<400> 6
   cgggatccag acatgataag atacattg 28
<210> 7
   <211> 44
   <212> DNA
   <213> Homo sapiens
<400> 7
   atagtttagc ggccgcaatg aatgcaattg ttgttgttaa cttg 44
<210> 8
   <211> 33
   <212> DNA
   <213> Homo sapiens
<400> 8
   tggcgcgcct atagggagac ccaagctggc tag 33
<210> 9
   <211> 38
   <212> DNA
   <213> Homo sapiens
<400> 9
   caatcatacc gtttaaacga accgcgggcc ctctagac 38
<210> 10
   <211> 59
   <212> DNA
   <213> Homo sapiens
<400> 10
   ggggacaagt ttgtacaaaa aagcaggcta tggaagacgc caaaaacata aagaaaggc 59
<210> 11
   <211> 54
   <212> DNA
   <213> Homo sapiens
<400> 11
   ggggaccact ttgtacaaga aagctgggtt tacacggcga tctttccgcc cttc 54
<210> 12
   <211> 407
   <212> DNA
   <213> Homo sapiens
<400> 12
   aagctttttg caaaagccta ggcctccaaa aaagcctcct cactacttct ggaatagctc        60
   agaggccgag gcggcctcgg cctctgcata aataaaaaaa attagtcagc catggggcgg       120
   agaatgggcg gaactgggcg gagttagggg cgggatgggc ggagttaggg gcgggactat       180
   ggttgctgac taattgagat gcatgctttg catacttctg cctgctgggg agcctgggga       240
   ctttccacac ctggttgctg actaattgag atgcatgctt tgcatacttc tgcctgctgg       300
   ggagcctggg gactttccac accctaactg acacacattc cacagctggt tctttccgcc       360
   tcagaaggta cctaaccaag ttcctctttc agaggttatt tcaggcc                     407